# EUROPEAN PATENT SPECIFICATION

(11) **EP 1 907 532 B1**
(45) Date of publication and mention of the grant of the patent: **29.09.2010**
(21) Application number: 06765082.0
(22) Date of filing: 21.07.2006
(51) Int. Cl.: C12N 5/071

(54) **BIOLOGICAL MATERIALS AND USES THEREOF**
BIOLOGISCHE MATERIALIEN UND VERWENDUNGEN DAVON
MATÉRIAUX BIOLOGIQUES ET LEURS UTILISATIONS

(30) Priority: 22.07.2005 GB 0515006
(43) Date of publication of application: 09.04.2008
(73) Proprietor: The University of Nottingham, Nottingham NG7 2RD (GB)
(72) Inventor: JOHNSON, Andrew, Institute of Genetics, Nottingham NG7 2UH (GB); CAMPBELL, Keith, Div. of Animal Physiology, Loughborough, Leicestershire LE12 5RD (GB); ALBERIO, Ramiro, Div. of Animal Physiology, Loughborough, Leicestershire LE12 5RD (GB)
(74) Representative: Wainwright, Jane Helen
(86) International application number: PCT/GB2006/002754
(87) International publication number: WO 2007/010287

(56) References cited:
- EP-A- 1 391 503
- JOHNSON ANDREW D ET AL: "Regulative germ cell specification in axolotl embryos: A primitive trait conserved in the mammalian lineage." PHILOSOPHICAL TRANSACTIONS OF THE ROYAL SOCIETY OF LONDON B BIOLOGICAL SCIENCES, vol. 358, no. 1436, 29 August 2003 (2003-08-29), pages 1371-1379, XP002399630 ISSN: 0962-8436
- HANSIS C ET AL: "Nuclear Reprogramming of Human Somatic Cells by Xenopus Egg Extract Requires BRG1" CURRENT BIOLOGY, CURRENT SCIENCE,, GB, vol. 14, no. 16, 24 August 2004 (2004-08-24), pages 1475-1480, XP004548528 ISSN: 0960-9822
- BACHVAROVA ROSEMARY F ET AL: "Gene expression in the Axolotl germ line: Axdazl, Axvh, Axoct-4, and Axkit" DEVELOPMENTAL DYNAMICS, vol. 231, no. 4, December 2004 (2004-12), pages 871-880, XP002399631 ISSN: 1058-8388 cited in the application
- MCGANN C J ET AL: "MAMMALIAN MYOTUBE DEFIFFERENTIATION INDUCED BY NEWT REGENERATION EXTRACT" PROCEEDINGS OF THE NATIONAL ACADEMY OF SCIENCES OF USA, NATIONAL ACADEMY OF SCIENCE, WASHINGTON, DC, US, vol. 98, no. 24, 20 November 2001 (2001-11-20), pages 13699-13704, XP001069019 ISSN: 0027-8424
- GURDON J B: "The use of Xenopus oocytes and embryos as a route towards cell replacement" JOURNAL OF BIOSCIENCES (BANGALORE), vol. 30, no. 1, February 2005 (2005-02), pages 11-14, XP002399632 ISSN: 0250-5991

## Description

The present invention relates to the reprogramming of a differentiated cell or cell nucleus and uses thereof of the reprogrammed cell and/or cell nucleus. In particular, the invention describes methods of reprogramming a differentiated cell to produce an embryonic stem cell-like cell.

Embryonic stem cells are pluripotent stem cells which are capable of generating any other cell type in the organism from which they are derived. Pluripotent cells have the ability to develop into all three embryonic tissue layers which in turn form all the cells of every body organ and is used to describe stem cells that can form any and all cells and tissues in the body. This differs subtly from totipotent cells which have the capacity to specialize into extraembryonic membranes and tissues, the embryo, and all postembryonic tissues and organs.

Differentiated cells are cells which make up the somatic tissues within an organism and are, under normal *in vivo* circumstances, generally considered to be restricted in their developmental potential. Differentiated cells may be either terminally differentiated, that is, they are developmentally arrested as a particular cell type (for example, neurons, myocytes and osteocytes), or they may retain the potential to give rise to a limited number of other cell types within a specific lineage (for example, a myeloid progenitor cell which can produce a variety of immune cells including basophils, eosinophils, and neutrophils).

Mammalian, and in particular human, embryonic stem cells offer the potential for the treatment and/or prevention of many human diseases or conditions. In particular, embryonic stem cells offer a means to generate a large range of human cell types, such cells being of great therapeutic value. However, currently in most developed countries there are ethical and practical issues surrounding the obtaining and use of mammalian, and in particular human, embryonic stem cells.

Embryonic stem cells are normally derived from a pluripotent population of cells in an embryo known as the inner cell mass (ICM) which is the population of cells from which an entire organism is derived. The ICM arises early in development, typically three to five days after fertilisation depending on the species in question. Using cloning procedures it is possible to produce embryos from any individual of a given species. Typically, in current cloning procedures the nucleus of a mammalian differentiated cell is transferred into an enucleated mammalian oocyte, usually from the same species in a process known as nuclear transfer (Campbell et al., 2005, Reprod Dom Anim 40:256-268).

Nuclear transfer is known to be difficult to perform and to have a low frequency of success (Campbell et al., 2005, Reprod Dom Anim 40:256-268). When successful, the transferred nuclear DNA is reprogrammed by the recipient oocyte and the oocyte can then be stimulated, by mimicking the effect of fertilisation, to form an embryo.

Usually the oocyte is stimulated into mitosis and either at the morula or blastocyst stage is implanted into the uterus of a 'foster' mother. This procedure has a very low success rate and produces very few viable embryos. In those cases where an embryo forms, the ICM may be isolated to provide embryonic stem cells which can be used in other applications.

When this process is applied to human, or mammalian, embryos it is referred to as therapeutic cloning, which has both practical and ethical considerations. In practical terms, obtaining embryonic stem cells requires the donation of eggs from a female, which is limited in practice as mammalian eggs are expensive to recover and very few eggs can be obtained from one female. In ethical terms and particularly in relation to humans, there are widespread objections to the generation of an embryo as an intermediate in another process, for example in therapeutic cloning where the embryo produced serves only as a donor of embryonic stem cells.

Hence, there is a need to develop pluripotent, embryonic stem cell-like cells which can be used in therapeutic procedures but that can be obtained without the cloning procedure, and in particular without using mammalian oocytes to produce embryos simply to provide embryonic stem cells.

The nuclei of differentiated cells have previously been successfully reprogrammed towards a pluripotent state by the production of viable fertile animals from cloning experiments with differentiated cells (Wilmut et al (1997) Nature 385:810-813; Polejaeva et al (2000) Nature 407:86-90). These procedures have very low efficiencies and success rates. In these procedures the differentiated nucleus was reprogrammed to pluripotency by nuclear transfer into an enucleated mammalian oocyte and exposure to factors within the mammalian oocyte which reactivated genes in the cell nucleus that conferred pluripotency to the cell via the creation of an embryo. In these cases all the manipulations were performed using material from the same species as the cell of interest.

Pluripotency in a cell may be identified by looking for the expression of a number of marker genes. In a human these genes include POU5F1 (encoding the transcription factor Oct-4), NANOG, Rex-1, Sox-2 and Tert (Ginis et al., 2004 Dev Biol 269:Pages 360-380). The skilled man will understand from the context in which POU5 (Oct-4) and NANOG, or any other genes or proteins are referred to whether it is the gene or the gene product which is being discussed.

Oct-4 is a transcription factor normally found and expressed in pluripotent cells, including embryonic stem cells, but it is not expressed in normal differentiated cells. The activation of Oct-4 is consistent with the cells being reprogrammed towards a state resembling pluripotency. Experimental elimination of Oct-4 results in the complete absence of an ICM in embryos, and the loss of an undifferentiated phenotype in embryonic stem cells which go on to differentiate into primitive ectoderm (Nichols et al (1998) Cell. 95:379-391).

Nanog is another transcription factor also found in pluripotent cells. In the absence of nanog expression embryonic stem cells lose their pluripotency (Chambers (2004) Cloning Stem Cells 6:386-391).

EP 1 391 503 describes methods of regrogramming a samatic cell by contacting the cell with cytoplasm from a xenopus cell, and also describes the subsequent assaying of the reprogrammed cell for the expression of embryonic markers.

Johnson et al (2003) Phil. Trans. Royal. Soc. Lon. 358, p 1371-9 discloses that axolotl may be useful as a model system due to axolotl being shown to possess a common mechanism of germ cell development with mammals.

There is a need for methods of producing embryonic stem cells or cells that resemble embryonic stem cells (so-called embryonic-stem cell like cells) without the creation of whole embryos in order to overcome both the practical and ethical considerations of the stem cell field. Hence, the present invention provides methods of re-directing the developmental potential of differentiated cells and/or their nuclei e.g. differentiated cells from mammals, to a more pluripotent state resembling that of an embryonic stem cell in a process known as reprogramming.

In a first aspect of the invention there is provided a method of producing a reprogrammed cell or reprogrammed cell nucleus, comprising exposing a permeabilised differentiated cell, or the nucleus of a permeabilised differentiated cell, at a temperature of between about 5°C and about 30°C, to a cell or cell extract thereof derived from an oocyte, egg, ovary or early embryo of a cold blooded vertebrate, wherein the cold blooded vertebrate is an axolotl or a sturgeon.

Preferably the oocyte, egg, ovary or early embryo cell or cell from which the cell extract is derived, expresses both Oct-4 in a highly conserved form and nanog.

Alternatively the method can be described as a method of reprogramming a differentiated cell to express nanog and/or express Oct-4 and/or be pluripotent comprising exposing a permeabilised differentiated cell, at a temperature of between about 5°C and about 30°C, to an oocyte, egg, ovary or early embryo, or an extract thereof, from a cold blooded vertebrate, wherein the cold blooded vertebrate is an axolotl or a sturgeon.

Preferably the oocyte, egg, ovary or early embryo cell or cell from which the cell extract is derived, expresses both Oct-4 in a highly conserved form and nanog.

Further alternatively the method can be described as the present invention provides a method of producing an embryonic stem cell-like cell, or a method of producing a reprogrammed cell nucleus, comprising contacting a permeabilised differentiated cell or the nucleus of a permeadilised differentiated cell, at a temperature of between about 5°C and about 30°C, with an oocyte, egg, ovary or early embryo, or an extract thereof, from a cold blooded vertebrate which is an axolotl or a sturgeon.

A reprogrammed cell is a cell which has had its normal differentiated function removed or altered. A reprogrammed cell nucleus is a nucleus whose normal differentiated function has been removed or altered.

A differentiated cell is a cell which has developed into a particular cell type with a specified function e.g. a nerve cell, or muscle cell. These cells under normal circumstances cannot generally develop to other cell types. In the context of this invention a re-differentiated cell is a cell which has been of one particular cell type and has been manipulated using the methods of the invention to form a stem cell and subsequently converted into a differentiated cell again. The re-differentiated cell may be of the same type or a different type of cell as in its original differentiation.

### Primitive Body Plan

Whether an organism has a primitive vertebrate body plan can be determined by considering one or both of two specific criteria pertaining to their skeletal structures. The first criterion relates to the rib structure and the second criterion relates to the pelvic bone. Primitive fish and primitive amphibians have similar skeletal structures, which is in marked contrast to the skeletal structure of organisms with so called "derived" skeletons (anuran (frog) skeletons; teleost skeletons).

An organism with a primitive vertebrate body plan, such as a lungfish or a salamander, has ribs which radiate laterally from a backbone. This is the primitive condition of the vertebrate skeleton which gave rise to the rib cage in mammals. By way of contrast in an organism, such as a teleost fish, which has a derived body plan, the ribs radiate dorso-ventrally, not laterally. This difference allows fish with a primitive vertebrate body plan to be distinguished from those without.

As an alternative to considering the ribs, an organism with a primitive vertebrate body plan can also be identified by considering the pelvic bone. In primitive fish pelvic appendages extend from the pelvic bone in the posterior region of the skeleton. In the skeletons of teleosts the pelvic bone is found in a much anteriorised position near or adjacent to or even fusing with the pectoral girdle, positioning the pelvic fins at about the half-way point of the fish's body or even anterior of that point, near the head. The primitive amphibian skeleton exemplified by the salamander (axolotl) retains a pelvic bone attached to the posterior region of the skeleton. Frogs in contrast have a highly derived and expanded pelvic girdle. In addition frogs have a much reduced number of vertebrae anterior to the pelvic girdle.

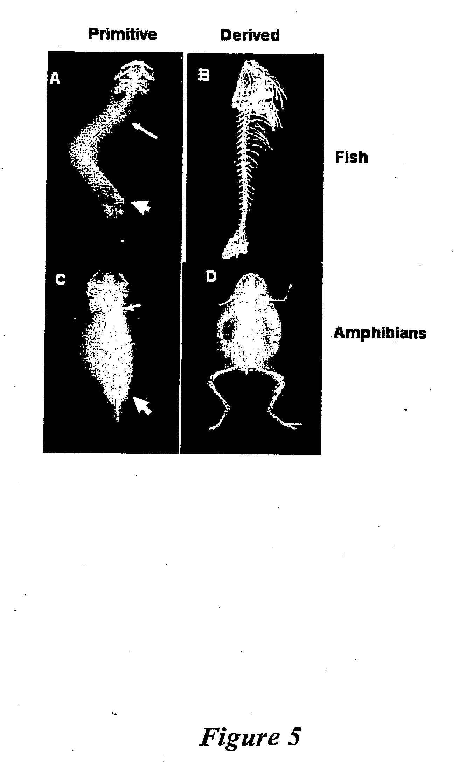

Figure 5 illustrates the retention of the primitive vertebrate body plan in fish and amphibians. The similarity in the skeletal structure between the primitive fish and primitive amphibian can be clearly seen, as can the difference in structure compared to that of the derived skeletons. The figure compares the skeleton of a lungfish, representing the primitive vertebrate body plan with that of a typical teleost skeleton, representing a derived vertebrate body plan. It also compares the skeleton of a salamander (axolotl) with a frog (*Xenopus laevis*).

The lungfish skeleton is shown from a dorsal view (from the back). Importantly, the teleost shows a side view, illustrating that the spinal projections/ribs radiate dorso-ventrally, not laterally. The amphibians are both dorsal views. In the fish the small arrows point to bones/ribs which project laterally from the backbone in the lungfish. This is the primitive condition of the vertebrate skeleton which gave rise to the rib cage. Note that the teleost ribs project ventrally, not laterally. This is a teleost innovation. The dorsal and ventral spinal projections which support the fins are also a teleost innovation. Primitive fish retain four limbs, which evolved into the four limbs of tetrapods, four-limbed land dwelling animals. Importantly, the large arrow points to the pelvic bone in the primitive fish and primitive amphibian. This is a feature that defines primitive fish, as it is lost in teleosts. The primitive vertebrate body plan is discussed in more detail in Johnson et al(2003) Evolution and Development 5:4, 414-431.

Sturgeon, turtle, lungfish, and mammals all share a similar embryology, as defined by gastrulation movements.

Therefore, the primitive vertebrate body plan can be identified, for example on x-rays, by the lateral and no dorsoventral projection of ribs and/or spinal projections. An alternative skeletal identification is the pelvic bone in fish.

### Germ cells without germplasm

In females a germ cell refers to an oocyte or a precursor cell to an oocyte.

An oocyte defines the female germ cell when in the ovary, and an egg defines the female germ cell after ovulation.

Germ plasm is a region of cytoplasm found in the egg, oocyte or embryo of some organisms which contains determinants that will give rise to the germ cell lineage. In animals without a germ plasm these same molecules are distributed throughout the egg cytoplasm, and possibly the GV (germinal vesicle) as well. These molecules, such as the products of the nanos, vasa and dazl genes, are typically RNA binding proteins, or the messenger RNAs that encode these, that are likely to be involved in the pluripotency reprogramming process since they are found in embryonic stem cells. It is considered that in organisms with a germ plasm these molecules are localized, and therefore probably in low abundance and relatively unavailable in oocytes, eggs, ovaries or early embryos, or extracts thereof, thus making organisms with a germ plasm unsuitable for reprogramming differentiated cells.

Axolotl and sturgeon are cold blooded vertebrates with a primitive vertebrate body plan that also has oocytes/eggs (germ cells) which do not contain germ plasm. The presence or absence of germ plasm may explain how certain organisms developed. Indeed by looking at the body plan of an organism it is possible to predict whether the oocytes/eggs in that organism will have a germ plasm. The hind limbs in particular, which define the posterior extreme of the body trunk, are crucial. In the absence of germ plasm the germ cells are derived from posterior-lateral mesoderm, at about the vertebral position defined by the pelvic bone and they are formed in response to extracellular signals exclusive to this posterior region of the embryo. These cells later develop into oocytes. In the presence of germ plasm the germ cells develop in a more anterior position and the resulting organism has a relatively anterior adult morphology. The oocytes, eggs, ovaries and early embryos of organisms with a germ plasm are less efficient at cell and nucleus reprogramming when compared to those without a germ plasm.

Organisms which display a primitive vertebrate body plan are understood not to have germ plasm in their germ cells. The retention of the primitive vertebrate body plan is believed to be a consequence of the absence of a germ plasm.

For the purposes of identifying animals whose oocytes/eggs contain germ plasm, the hindlimbs, defining the posterior extreme of the body trunk are crucial. In the absence of germ plasm germ cells are derived from posterior-lateral mesoderm, at about the vertebral position defined by the pelvic bone. Animals whose eggs contain germ plasm do not require the extracellular posterior signals required to produce germ cells as the cells are specified by material supplied by the egg, not from extracellular signals. Therefore, in most animals with germ plasm, such as frogs and teleost fish, the animals have a relatively anterior adult morphology. The oocytes and eggs of these animals are not as useful for reprogramming.

It has been shown that the absence of germ plasm in mammals, once considered to be a mammalian innovation, has been conserved through a specific lineage of species that retain primitive embryological characteristics, which as a consequence result in the retention of a primitive vertebrate body plan in this lineage of animals. In the embryos of mammals and cold-blooded vertebrate species whose embryos retain primitive characteristics, and retain a primitive adult vertebrate morphology the stem cells that give rise to the germ line (sperm and eggs), known as PGCs, are derived from pluripotent precursors that arise early in development. Whereas in most experimental, non-mammalian, organisms such as *Xenopus* or zebrafish (a teleost) the PGCs are formed by an entirely different mechanism than in mammals, and pluripotent precursors equivalent to those of mammals are never formed. In *Xenopus* and zebrafish the germ cells are segregated very early from somatic cells by the unequal distribution of germ plasm, containing germ cell determinants. The cells that inherit the germ plasm then become the PGCs, and do not give rise to somatic cells.

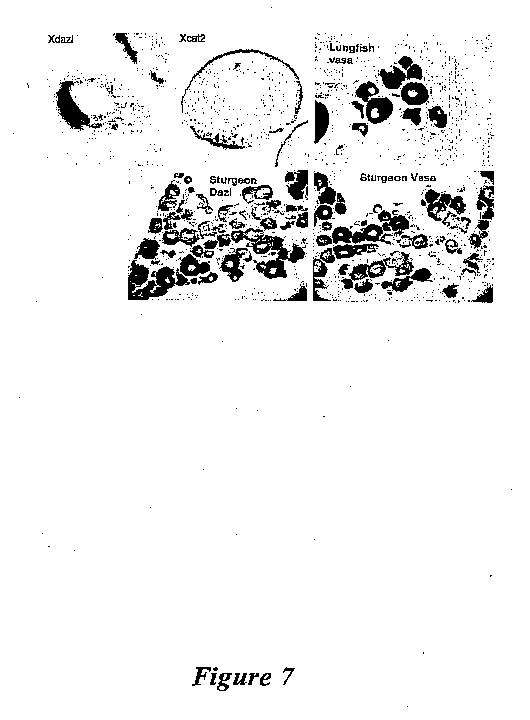

Figure 7 illustrates the distribution of germ plasm in oocytes from a number of different organisms. The RNAs coding for Xdaz1 and Xcat2 in Xenopus oocytes are shown, and the RNA coding for vasa is shown in lungfish oocytes. RNA coding for dazl and vasa are shown for sturgeon oocytes. Sections from oocytes were reacted with probes specific to these molecules and the probe was detected using alkaline phosphatase conjugated antibodies and colour detection by standard methods. Xdaz1 and Xcat2 RNAs are localized within the cytoplasm, indicative of germ plasm. Vasa RNA in lungfish oocytes and vasa and dazl RNA in sturgeon oocytes, are uniformly distributed, indicative of the absence of germ plasm.

It is now believed that the mechanism to produce PGCs in mammals is present in some lower animals, but only in those species that meet specific criteria. These species can be identified on the basis of their body plan. Species that meet the criteria, such as axolotl, have PGC development similar to that in mammals, and a similar complement of genes that govern germ cell development, such as the genes encoding Oct-4, and nanog and are therefore able to reprogram differentiated cells, and in particular mammalian differentiated cells. It is therefore possible to accurately predict that the oocytes of certain species which meet the aforementioned criteria will have reprogramming potential equivalent to that of axolotls. Species that do not meet these criteria will not have the same complement of conserved pluripotency genes and thus will not have equivalent reprogramming capability. The species meeting the criteria have a primitive vertebrate body plan which is believed to be a consequence of the absence of a germ plasm. Importantly, since Oct-4 and nanog are required to produce PGCs in the absence of germ plasm, the retention of Oct-4 and nanog is believed to be a consequence of having no germ plasm.

Adults are a product of their embryology. Therefore, the adult body plan is conserved because the embryology is conserved. What constrains the embryology, preventing the major changes seen in frogs and teleosts, is the mechanism for producing germ cells. In animals without germ plasm the PGCs have a more tenuous existence, they can be wiped out if the signals required for their production are altered, as would happen with a major change in embryological cell movements. If germ plasm is present, the PGCs are refractory to these changes in signals, because they form no matter what. Thus, constraints on the embryology are lifted, the cell movements of embryology change, and this is why animals with germ plasm are capable of having an altered morphology from the primitive body plan. Without germ plasm this couldn't happen. Sturgeon, turtle, lungfish, and mammals all share a similar embryology, as defined by gastrulation movements.

### Expression of Oct-4 and/or nanog

Cold blooded vertebrates retaining a primitive vertebrate body plan may also have oocyte, egg, ovary or early embryo cells which express Oct-4 and/or nanog.

Oct-4 and/or Nanog expression may be determined by assaying for mRNA encoding the protein or for the protein product itself. Examples of methods of assays to do this include RT-PCR method to assay mRNA described in Makin et al. technique 2 p295-301 (1990) or antibody assays for the protein product.

In order for Oct-4 and/or nanog in the oocyte, egg, ovary or early embryo cells to be considered highly conserved it must share at least 69% amino acid identity with the DNA binding domains (DBD) of the human transcription factor Oct-4 or the human nanog protein, respectively. In the case of the axolotl Oct-4 protein, AxOct-4, 73% of the amino acids are identical to the mouse Oct-4 DBD, and 75% are identical to the human Oct- 4 DBD. The closest proteins from zebrafish and Xenopus, encoded by the genes ZPOU-2 and XLPOU91, and which do not confer the ability to reprogram differentiated cells respectively, are 63% identical, less than the required 69% identity. The identity and activity of the functionally equivalent Oct-4 genes can be tested using standard methods known to the skilled man such as those described in the example.

The amino acid identity referred to between the human Oct-4 DBD and that of a cold blooded vertebrate allows for conservative changes in the amino acids which do not alter the polarity or charge of the amino acid residue. Examples of conservative changes are well known to those skilled in the art, and include, for example, substitution of one hydrophobic residue such as isoleucine, valine, leucine or methionine for another, or the substitution of one polar residue for another, such as arginine for lysine. Preferably, when determining the amino acid identity between two protein sequences conservative amino acid changes are considered to be identical amino acids. Therefore, a conservative amino acid change in amino acid sequence will not affect the percentage amino acid identity between to two sequences.

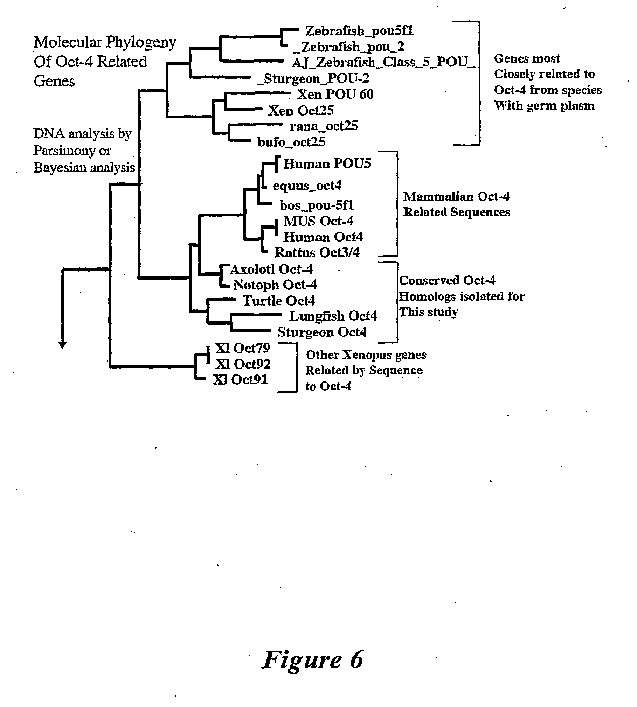

To further support the understanding discussed above, the gene encoding Oct-4 in various species was compared, and those sharing the identified morphological features were shown to have more closely related Oct-4 encoding genes than species lacking the morphological features. In these experiments the Oct-4 encoding genes were isolated from newt (Notopthalmus viridens), the gulf sturgeon (Asciperser oxyrhynchus), the African lungfish (Protopterus annectans) and the Red Eared Slider (a turtle, T.scripta) all of which fulfil the criteria described for primitive morphology. In Figure 6 the DNA sequences of Oct-4 encoding related genes isolated from these species are compared with the most closely related gene sequences isolated from other species that are part of the public database and a pseudo phylogenetic tree is illustrated designed to track the evolutionary history of the Oct-4 encoding gene. Under normal circumstances a gene sequence evolution tracks the evolutionary relatedness of the animals (i.e. the species phylogeny). Therefore any sequence of a related gene would be expected to be more similar between salamanders (axolotl and newt) and frogs (Xenopus, Bufo, rana) because of their more recent divergence from a common amphibian ancestor, than they would be to the equivalent gene from another species, such as, fish, reptiles or mammals, from which they are evolutionarily more distant. The same logic holds for all fish compared to higher order species such as mammals and amphibians. However, in contrast to conventional phylogenetic predictions the analysis in Figure 6 shows that salamanders (i.e. axolotl), newts, sturgeon, lungfish and turtles each contain a gene highly related to the Oct-4 gene of the mouse and human Oct-4 genes, the equivalent gene is not found in their most closely related sister groups i.e. frogs, zebrafish etc. The gene sequence relationships shown in Figure 6 are not obvious to the scientific community, and would not be predicted on any conventional view of evolution.

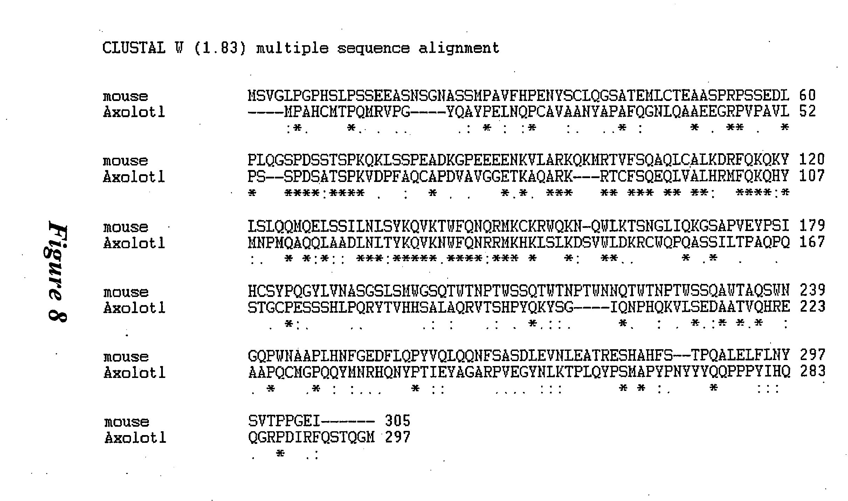

The Oct-4 gene (Axoct-4) in the axolotl (the salamander species *Ambystoma mexicanum*) is more highly related to the mammalian Oct-4 gene than any other gene in the database, including several genes from Xenopus that are related to Oct-4, and can rescue ES cells, in some cases, such as XLPOU91, better than the axolotl Oct-4 gene (Morrison and Brickman, 2006). (Figure 8 shows comparison of axolotl and mouse sequences). This result suggests that the Axoct-4 gene is a true ortholog (i.e. a gene related by ancestry and function) of the mammalian Oct-4 gene (this is also supported by the expression pattern (Bachvarova et al (2004) Developmental Dynamics 231:871-880). The greater number of Xenopus genes result from duplications of an ancestral Oct-4 sequence, more similar to Axoct-4, and subsequent subfunctionalization of mechanism (Prince and Pickett, 2002; Nat Rev. Genet.: 3; 827-37). This result suggests that the axolotl oocytes are biochemically more similar to the oocytes of mammals than are the oocytes of *Xenopus* which do not contain a true Oct-4 ortholog.

In mammals, Oct-4 is required to produce PGCs. This is not the case in Xenopus, meaning that any Oct-4 equivalent genes are not a true orthologs of the mammal Oct-4. The expression pattern and homology of mechanism of Oct-4 in axolotl now suggest Oct-4 is required to produce PGCs. This observation may contribute to why axolotl oocytes have a greater capacity to reprogram mammalian differentiated cells to pluripotency than *Xenopus* cells, the axolotl oocytes behaving more like mammalian oocytes in reprogramming capacity. Also, since the absence of germ plasm also makes these oocyte/eggs more similar to mammals, this is likely to also be involved in the superior reprogramming capacity of axolotls compared to *Xenopus.* The absence of a germ plasm may allow other factors such as Nanos, vasa and dazl which are all RNA binding proteins associated with germ plasm to be more accessible for reprogramming.

Having demonstrated that axolotls, which have an Oct-4 gene more closely related to mammalian Oct-4 genes than the Oct-4 gene of frogs , it is understood that other organisms with an Oct-4 gene more closely related to the mammalian Oct-4 gene will also be able to reprogram mammalian differentiated cells. Also, the expression pattern of Axoct-4 is equivalent to that of early mammalian embryos. Thus sturgeon and lungfish, other salamanders, and the "primitive fish" will all have a reprogramming capacity equivalent to axolotl, and far greater than that of *Xenopus,* other frogs, or teleosts.

Preferably the reprogrammed cell is an embryonic stem cell-like cell.

The term "embryonic stem cell-like cell" is used herein to refer to a differentiated cell that has been reprogrammed to exhibit a property of an embryonic stem cell, or a cell containing a reprogrammed differentiated nucleus which exhibits a property of an embryonic stem cell. An embryonic stem cell-like cell may include one or more of, but not limited to, the following properties: proliferation without transformation; continuous proliferation; self renewal and capacity to generate a wide range of tissues; the ability to differentiate into either the same or a different cell type than the original differentiated cell (pluripotency); when compared with these same parameters in the cell prior to being de-differentiated or reprogrammed.

Preferably, the reprogrammed cell, and/or the reprogrammed cell nucleus, expresses Oct-4. Alternatively the reprogrammed cell, or the reprogrammed cell nucleus, expresses only nanog. The reprogrammed cell, or the reprogrammed cell nucleus may also be pluripotent.

Conveniently the reprogrammed cell, and/or the reprogrammed cell nucleus produced according to any method of the invention may express both Oct-4 and nanog and/or other markers of pluripotency. Other markers of pluripotency include Sox-2, Rex-1, and TERT.

Whether a cell is pluripotent can be identified by the presence of pluripotent properties, that is that the cell can be stimulated to differentiate into almost any cell type in the organism from which it is derived. Differentiation of pluripotent cells can be induced by exposure of the pluripotent cell to a progenitor medium and/or certain growth factors Lanza, 2004. Handbook of Stem Cells: Embryonic/Adult and Foetal Stem Cells.

Preferably the Oct-4 and/or the nanog in the cold-blooded vertebrate oocyte, egg, ovary or early embryo cell or cell extract are highly conserved in comparison to the human form, by this we mean the Oct-4 in the cold-blooded vertebrate has at least 69% amino acid identity in the DNA binding domain with the human transcription factor Oct-4. Preferably with the nanog gene this would be at least 69% and most preferably 75% identity with the homeo domain shared with the human nanog protein,

Advantageously the Oct-4 and/or the nanog in the cold blooded vertebrate oocyte, egg, ovary or early embryo cells has at least 69% amino acid identity with the DNA binding domains (DBD) of the human transcription factor Oct-4 or the human nanog protein, respectively.

Amino acid identity can be measured using ClustalW (Thompson et al. (1994) Nucl. Acids. Res., 22 p4673-4680, or any alternative amino acid sequence comparison tool. The clustalW method can be used with the following parameters:
Pairwise alignment parameters - method:accurate
Matrix: PAM, Gap open penalty 10.00, Gap extension penalty: 0.10;
Multiple alignment parameters - Matrix: PAM, Gap open penalty: 10.00, % identity for delay: 30; Penalize end gaps: on. Gap separation distance 0, Negative matrix: no, Gap extension penalty: 0.20, Residue -specific gap penalties: on, Hydrophilic gap penalties: on, Hydrophilic residues: GPSNDQEKR. Sequence identity at a particular residue is intended to include identical residues which have simply been derivatized.

Alternative parameters may also be suitable.

Nucleotide sequence identity can also be measured using ClustalW (Thompson et al (1994)) using the following parameters:
Pairwise alignment parameters - Method: accurate,
Matrix: IUB, Gap open penalty: 15.00, Gap extension penalty: 6.66;
Multiple alignment parameters - Matrix: IUB, Gap open penalty: 15.00,
% identity for delay: 30, Negative matrix: no, Gap extension penalty: 6.66, DNA transitions weighting: 0.5.

### Alternative parameters may also be suitable.

Preferably, any nucleotide sequence encoding a conserved Oct-4 and/or nanog has more than 69% e.g. 75%, 80%, 90% or 95% identity to the human Oct-4 and/or nanog sequences (according to the test described above) or a sequence which hybridizes to human Oct-4 and/or nanog sequences under wash conditions of 0.1 x SSC, 65°C (wherein SCC = 0.15 M NaCl, 0.015M sodium citrate, pH 7.2) which encodes a functionally equivalent protein to human Oct-4 and/or nanog.

Polypeptides of the invention include both full-length and fragments. Such polypeptides may be prepared by any conventional means.

Functionally equivalent proteins or protein fragments refer to proteins and/or fragments having at least 69% sequence identity and retaining the same function as human Oct-4 and/or nanog. Such function can be tested by any of the methods described herein.

"Nucleic acid molecules" according to the invention include single and double stranded DNA, RNA, cDNA. Derivatives of nucleotide sequences capable of encoding functionally-equivalent polypeptides may be obtained using any conventional method well known in the art.

The group of organisms predicted on the basis of body plan to lack a conserved Oct-4 gene, including all frogs, all teleost fish, all birds and the majority of reptiles is far larger than the group that retain a primitive body plan and that retain a conserved *Oct-4* gene (including urodele amphibians) and a gene encoding an ortholog of nanog. The following lineages retain a conserved vertebrate body plan:
Salamanders (axolotl or notopthalamus)
Turtles
Lizards
Crocodilians
Hyperotreti (hagfish);
Hyperoartia (lamprey);
Chondrichthyes (sharks, rays, skates, chimeras);
Chondrostei (bichirs, sturgeons, paddlefish);
Semionotiformes (gars);
Amiiformes (bowfins)
Dipnoi (lungfish); and
Coelacanthimorpha (coelacanths).

It is a particular advantage in that in a large proportion of the organisms mentioned it is relatively straightforward to obtain the oocytes, eggs, ovaries or early embryos cells or cell extracts thereof, and the material is abundant. Amphibians, and bony fish can have eggs several thousand times the volume of a mammalian egg and in quantities far exceeding mammalian egg tissue e.g. the sturgeon (from which caviar is obtained) can produce 15-25% of its bodyweight in egg cells. The recovered material can be stored and used as required.

By way of contrast, mammalian oocytes, eggs, ovarian material and/or early embryos are scarce and difficult to obtain, furthermore the material is very limited in amount.

The oocyte, egg, ovary or early embryo cells and cell extracts from *Xenopus laevis* or any other frog or any teleost fish are not suitable for use in any method of the invention. Frogs and teleost fish (both cold blooded vertebrae) are examples of amphibians and fish that do not retain a primitive body plan, their oocytes are known to contain germ plasm, and their Oct-4 protein is not highly conserved when compared to the human Oct-4 protein.

Preferably the oocyte, egg, or early embryo cell extract comprises material from the nucleus or germinal vesicle (GV) of the oocyte, egg, or early embryo cell.

The nucleus and GV contain specific transcription factors Oct-4 and Nanog.

The RNA encoding germ cell specific RNA binding proteins that are located in the germ plasm in frog sna teleosts Dazl, VASA, and Nanos are distributed uniformly in the cytoplasm (Johnson et al., 2001, 243:402-415; Dev,. Biol.; Bachvarova et al., Dev. Dyn. 231, 871-880) and are capable of maintaining pluripotency or germ cell specification in germ cells with germ plasm but are not capable of reprogramming cells.

Exposure of the differentiated cell with the nucleus or germinal vesicle of the oocyte, egg, or early embryo of an axolotl or sturgeon according to the invention, or an extract thereof, is achieved by injecting a permeabilised differentiated cell into the oocyte, egg, ovary cell or early embryo cell, or incubating a permeabilised differentiated cell with an extract of the oocyte, egg, ovary or early embryo.

It is envisaged that the permeabilised differentiated cell allows factors in the oocyte, egg, ovary or embryo, or extract thereof, to pass into the cell and reprogram it, preferably mitochondria or the nucleus from the oocyte, egg, ovary cell or embryo cell cannot pass into the permeabilised cell and thus there is no exchange of genetic material. Permeabilisation of the differentiated cell can be achieved by any method well known in the art such as treating the cell with Triton-X-100, digitonin or saponin.

Preferably, after contact with the oocyte, egg, ovary or early embryo, or an extract thereof, of an axolotl or sturgeon according to the invention the reprogrammed cells are recovered by centrifugation onto a microscope slide or culture dish, using techniques well known in the art.

Preferably, in any method of the invention for reprogramming a differentiated cell nucleus, a differentiated cell nucleus may be contacted with an oocyte, egg, ovary or early embryo, by using well known nuclear transfer techniques which will be readily apparent to the skilled man (see refs discussed above). Such techniques include injection of the differentiated nucleus into an enucleated oocyte or egg; or fusion of a differentiated cell with an enucleated oocyte or egg.

Cell based work has the advantage that the reprogrammed cell is entirely contained within the cold-blooded vertebrate cell.

Alternatively, the differentiated cell nucleus may be incubated with an extract of oocyte, egg, ovary or early embryo.

Use of an extract has the advantage that it avoids the manipulation necessary to inject a differentiated cell or nuclei into an intact oocyte, egg or early embryos in order for factors in the oocyte, egg, ovary or early embryo to act on the differentiated cell or nuclei and cause its reprogramming. Using extracts also makes it easy to retrieve the material and allows thousands to millions of cells or nuclei to be reprogrammed at once. Furthermore, oocyte, egg, ovary and early embryo extracts of a cold blooded vertebrate according to the invention can be produced in large amounts and can be stored for use as dictated by convenience.

Preferably an extract of whole ovary is used. By using whole ovary extract there is no need to separate the cell components, which might be impossible with some species of cold blooded vertebrate. Alternatively, the raw material for the extract may be oocytes liberated from ovarian stroma by conventional techniques, for example 0.2% collagenase digestion.

A differentiated cell refers to a cell that has achieved a mature state of differentiation. Typically a differentiated cell is characterised by the expression of genes that encode differentiation-associated proteins in a given cell. For example, the expression of myelin proteins and the formation of myelin sheath in glial cells is a typical example of terminally differentiated glial cells. Differentiated cells are either unable to differentiate further or can only differentiate into specific cells in a particular cell lineage.

Preferably, the differentiated cell used in any method of the invention is a eukaryotic cell. Preferably the differentiated cell is obtained from a mammal. Most preferably the cell is human.

Preferably, in any method of the invention, the differentiated cell or nuclei thereof is exposed to the oocyte, egg, ovary or early embryo cell or cell extract thereof, at a temperature between about 5°C and about 30°C, more preferably between about 5°C and about 21 °C. More preferably the differentiated cells or nuclei are contacted with the oocyte, egg, ovary or early embryo, or extract thereof, at a temperature consistent with the body temperature of the organism from which the oocyte, egg, ovary or early embryo cell or cell extract thereof, is derived. As the skilled person will appreciate, cold blooded animals don't have a body temperature of their own per se, their bodies are the temperature of their environment. More preferably the contact temperature is about 18°C. or that of the environment in which the cold blooded vertebrate normally resides.

Preferably, a reprogrammed cell nucleus according to the invention expresses genes which are markers of pluripotency. Preferably the pluripotency marker genes include the gene encoding Oct-4 and nanog. The Oct-4 gene may be used as a marker if demethylation of the Oct-4 promoter occurs.

In a second aspect of the invention there is provided a method of producing a re-differentiated cell comprising:
(a) producing a reprogrammed cell as described in the first aspect of the invention; and
(b) re-differentiating the reprogrammed cell into a differentiated cell of the same type, or a different type, to the differentiated cell from which it is derived.

Preferably, the re-differentiation is effected using a progenitor medium. Once generated, the reprogrammed differentiated cell can be cultured in the presence of particular growth factors and other signalling molecules (progenitor medium) that induce their differentiation into particular cells types. Different progenitor media are required to produce different cell types.

The method of the invention allows pluripotent reprogrammed cells to be obtained from differentiated cells of an individual, these reprogrammed cells can then be differentiated into a cell type required to treat that patient.

Different cell/tissue types are needed for subsequent use in different medical conditions.

For example, haematopoietic stem cells could be used to treat individuals suffering from leukaemia. Neural progenitor cells could be used to treat individuals suffering from neurodegenerative disorders, such as Alzheimer's or Parkinson's disease. Skin cells could be used for grafts in cases where an individual has suffered severe burns or scarring.

Stem cells may be used for the generation of organs and tissues for transplantation and may provide a promising alternative therapy for diabetes, liver disease, heart disease and autoimmune disorders to name just a few. The main problems associated with transplantation are the lack of donors and the potential incompatibility of the transplanted tissue with the immune system of the recipient.

That is, the immunorejection of the transplanted tissue as the recipient views the transplant as foreign. Embryonic stem cell-like cells may be derived from a patient in need of a transplant and then used to a produce tissue or an organ for transplantation into the same patient. This removes any problems of tissue incompatibility and immunorejection. Thus there is great therapeutic potential in being able to generate pluripotent embryonic stem cell-like cells, in particular where they are genetically identical to those of the patient.

In a third aspect of the invention there is provided a kit for reprogramming a differentiated cell, or for reprogramming the nucleus of a differentiated cell, comprising a cell or cell extract thereof derived from an oocyte, egg, ovary or early embryo of a cold blooded vertebrate, wherein the cold blooded vertebrate is an axolotl or a sturgeon;
instructions to use the oocyte, egg, ovary or early embryo, or extract thereof, and a progenitor medium to effect the further step of re-differentiation of the reprogrammed cell.

Preferably the oocyte, egg, ovary or early embryo cell or cell from which the cell extract is derived, expresses both Oct-4 in a highly conserved form and nanog.

Optionally the kit further comprises one or more differentiated cells to be reprogrammed.

### Preferred Embodiments

Examples embodying certain preferred aspects of the invention will now be described with reference to the following figures in which: -
**Figure 1** - shows the results of RT-PCR experiments looking for the expression of β-actin, nanog and Oct-4 gene in embryonic stem cells (ES), permeabilised foetal fibroblasts (PFF), and PFFs injected into the GV of either axolotl (PFFA) or *Xenopus* oocytes (PFF);
**Figure 2** -Axananog expression profile over 40 developmental stages as defined in Bordzilovskaya et al; 1989. Developmental stage series of axolotl embryos. In Developmental Biology of the Axolotl. (ed. J. B. Armstrong and G. M. Malacinski), pp. 210-219. New York: Oxford University Press. EC = Early Cleavage, LC = Late Cleavage;
**Figure 3** - shows that the incubation of PFF in extracts from axolotl ovary diminishes the amount of methylated DNA. Differentiated cells were either untreated (Control) or incubated in extracts prepared from axolotl ovary (AxOvEx) for 3 hours (3h). Cells were stained with antibodies for methylated DNA;
**Figure 4** - shows that the incubation of PFF in axolotl ovary extracts (AvOvEx) eliminates H3K9 staining;
**Figure 5** - depicts a comparison of skeletons representing evolutionarily conserved primitive and derived adult body plans in fish and amphibians. A. Lungfish skeleton shown from dorsal view. B. Teleost fish shown from a side view. C. Salamander (axolotl) skeleton showed from dorsal view. D. Frog (*Xenopus*) skeleton showed from dorsal view. Small arrows show ribs. Large arrows point to pelvic bones;
**Figure 6** - shows a phylogenetic analysis of class V POU domain transcription factors (Oct-4 like genes). Genes were isolated from the ovaries of the species and compared by parsimony to determined the most closely related sequences. Groups within the same cluster are the most closely related; and
**Figure 7** - shows examples of the distribution of germ cell specific RNAs in the cytoplasm of cold blooded vertebrates as indictors of the presence or absence of germ plasm.
**Figure 8** - Amino acid alignment of mouse and axolotl Oct-4 using Clustal-W.
**Figure 9** - Amino acid alignment of nanog from mouse, rat, human, cow, dog, opossum, chick and axolotl using Clustal-W.
**Figure 10** -A -Axolotl germinal vesicle isolated two days after injection of mammalian cells B - Oocytes injected with human primary fibroblasts were collected at the indicated days and the expression of Actin, Nanog and Oct-4 was analysed by RT-PCR.
**Figure 11** - RT-PCR gel showing Nanog and β-actin expression after exposure of differentiated cells to Xenopus, axolotl and/or sturgeon extracts.
**Figure 12** - Activation of a reporter gene in which a nanog binding site is present.

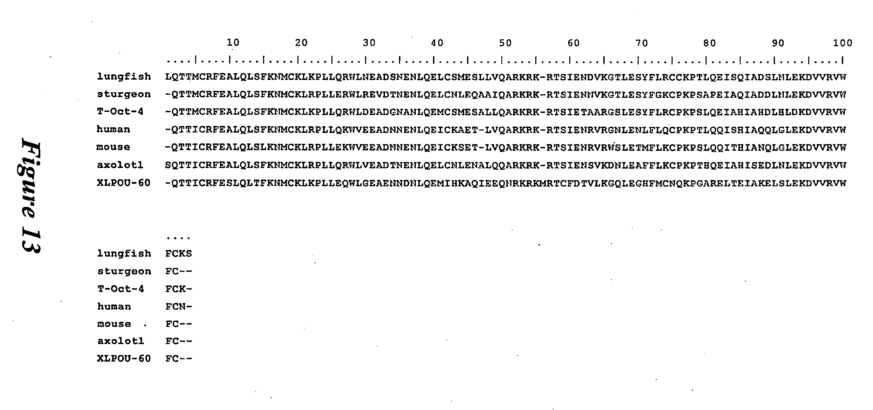

**Figure 13** - Oct-4 DNA binding domain sequence comparison using Clustal W - highly conserved species.

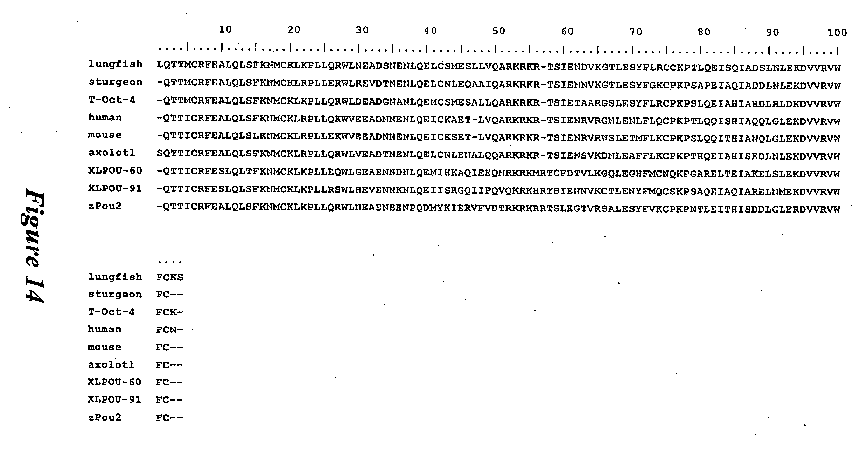

**Figure 14** - Oct-4 DNA binding domain sequence comparison using Clustal W - highly conserved species and non-related species such as Xenopus (XLPOU-60 and XLPOU-91) and Zebrafish (zPou2).

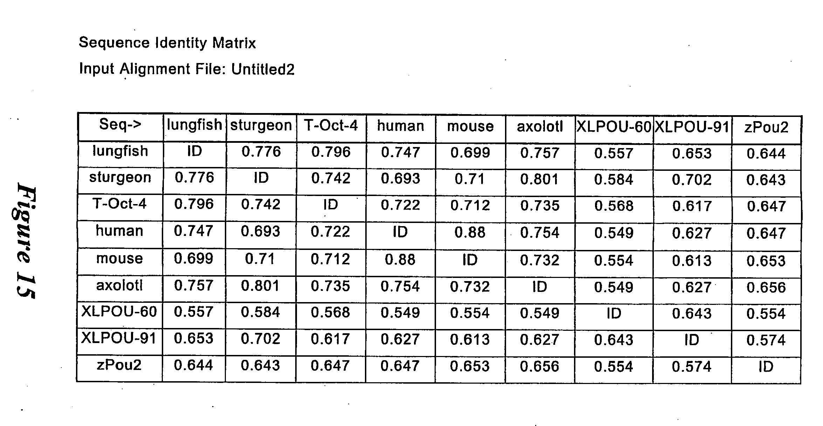

**Figure 15** - Table showing sequence identity of highly conserved Oct-4 genes compared to non-conserved Oct-4 equivalents in Xenopus and Zebrafish
**Figure 16** **-** DNA methylation in cells incubated in axolotl oocyte extracts (AOC) was assessed by staining for 5-methylcytosine. Treated cells were sorted by FACS (Fluorescence activated cell sorter) after 1 and 3 hrs of treatment. In addition a group supplemented with apyrase (ATPase inhibitor) was included to show that the process is energy dependent. AP: after permeabilization control.
**Figure 17** - Trimethylated Histone H3 Lysine 9 (TriH3K9) in cells incubated in axolotl oocyte extracts was assessed by staining TriH3K9 with a specific antibody. Treated cells were sorted by FACS after 1 and 3 hrs of treatment incubation in extracts. In addition, a group supplemented with apyrase (ATPase inhibitor) was included to show that the process is energy dependent. AP: after permeabilization control.

### Example 1 - The Ability of Oocytes from Axolotl to Reprogram Differentiated Mammalian Cells

To demonstrate the ability of oocytes from axolotl to reprogram differentiated mammalian cells, mouse foetal fibroblast cells were injected directly into the GV (germinal vesicle) of *Xenopus* (PFFX) and axolotl (PFFA) oocytes, using a method similar to that of Byrne *et al* (2003) Curr Biol 15:13(14) 1206-13 who used only *Xenopus.*

Cultured mouse foetal fibroblasts were permeabilised by treatment with a mild detergent (digitonin) following the method of Alberio et al (2005) Exp Cell Res. 307(1):131-41. When permeabilised the molecules from the GV can diffuse into the differentiated cell or PFF (permeabilised foetal fibroblast). Approximately 50 to 200 PFF cells were injected per GV, and the injected oocytes were incubated overnight at 21°C. At that time the GV was dissected from the oocyte and those which contained PFF were considered further. RNA was extracted from the GV and reverse transcribed into cDNA and PCR was used to detect the expression of pluripotency genes from the PFF. More particularly PCR was used to determine the expression of genes encoding nanog and Oct-4. β-actin levels were determined as a control.

The above described method has the advantage over known methods in that it concentrates the RNA expressed by the mammalian differentiated cells and leads to a greater sensitivity in detecting mammalian transcripts. Since the aim is to detect mammalian RNA, which is a very tiny fraction of the total RNA, the simple isolation of the GV provides an enormous purification step, and improves sensitivity accordingly.

### Results

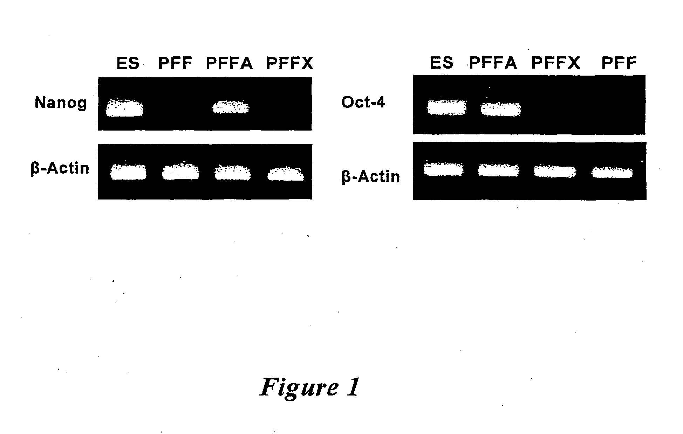

RT-PCR was performed to detect the expression of the pluripotency marker genes encoding Oct-4 and nanog in the PFF cell. Expression of β-actin was also studied as a control. β-actin is expressed in all cells at all times and serves as a positive control to ensure the efficiency of the assay procedure. Figure 1 shows the results of the RT-PCR reactions when run on an agarose gel. As the skilled man will be aware the presence of a white band indicates the presence of cDNA, the intensity of the band being indicative of the amount of cDNA (gene specific product).

The abbreviations used in Figure 1 are as follows:
ES - mouse embryonic stem cells (available from Chemicon)
PFF - mouse permeabilised foetal fibroblasts - prepared according to the method of Alberio et al (2005) Exp Cell Res. 307(1):131-41.
PFFA - PFF injected in axolotl oocyte GVs.
PFFX - PFF injected in *Xenopus,* oocyte GVs.

As can be seen from Figure 1 all the samples studied strongly express the gene for β-actin, which demonstrates that RNA from cells from each treatment is detectable by RT-PCR and that constitutive high level gene expression is detectable from cells regardless of treatment. The nanog gene encoding nanog and the gene encoding Oct-4 are expressed in the ES cells which is expected because these cells are pluripotent. Oct-4 and nanog (markers of pluripotency) are not expressed in the normal untreated PFF cells because these cells are differentiated fibroblasts which are not pluripotent. When the PFF cells were incubated overnight in the GV of axolotl oocytes (PPFA) expression of Oct-4 and nanog was detected. When the PFF cells were incubated in the GV of *Xenopus* oocytes (PFFX) overnight no expression of Oct-4 or nanog could be detected. After two days of incubation of the PFF cells in the GV of *Xenopus* oocytes low levels of Oct-4 expression was detected but still no nanog expression (data not shown). The Oct-4 expression detected after 2 days was only observed by a vast increase in the detection capability of the RT-PCR assay, that is, by increasing the cycles of replication from 30 to 60 rounds. Each additional round of PCR is effectively a doubling in sensitivity, so an increase to 60 rounds represents an increase of 2 to the 30^{th} power in sensitivity. Even under increased sensitivity conditions nanog expression could not be detected.

To conclude, axolotl oocytes induce on contact with permeabilised mammalian differentiated cells robust expression of the pluripotency marker genes encoding Oct-4 and nanog within 18 hours of incubation.

This is in comparison to *Xenopus* oocytes which although after several days can induce low levels of expression of Oct-4, no expression of nanog can be detected. Thus axolotl oocytes are much more effective at reprogramming the expression of pluripotency marker genes in mammalian differentiated cells than are *Xenopus* oocytes. Accordingly axolotl oocytes are much more effective at reprogramming differentiated cells to embryonic stem cell-like cells.

In this, and subsequent examples, the expression of a gene is determined by assaying for RNA encoded by the gene.

### Example 2 - Axolotl and Sturgeon compared to Xenopus

### Materials and Methods

### Preparation of egg, oocyte and ovarian extract

*Xenopus* oocyte and egg extracts were prepared according to Hutchison et al., (1988) Development, 103, 553-566. Unfertilised *Xenopus* eggs were collected from mature females and incubated in dejellying solution (20mM TrisHCl pH 8.5, 1 mM DTT and 110 mM NaCl) for 5 min. After dejellying, the eggs were rinsed three times in 0.9% NaCl saline and twice in ice-cold extraction buffer (20 mM Hepes, pH 7.5, 100 mM KCl, 5 mM MgCl₂, 2 mM β-mercaptoethanol) containing protease inhibitors (3 µg ml-1 leupeptin, 1 µg ml-1 pepstatin and 1 µg ml-1 aprotinin). The eggs were packed into 10-ml centrifuge tubes and excess buffer was removed before centrifugation at 10,000 g for 10 min at 4°C. The cytoplasmic layer was removed and supplemented with 50 µg ml-1 cytochalasin B before centrifugation at 100,000 g for 30 min at 4°C. The cleared cytoplasm was supplemented with 10% glycerol and snap frozen in liquid nitrogen in 100-200 µl aliquots. Oocyte extracts were prepared from mature ovaries by digesting follicle cells for 2 hrs at 25°C using 1 mg/ml collagenase in OR2 media (82.5 mM NaCl, 2.5 mM KCl, 1 mMCaCl₂, 1 mM MgCl₂, 1 mM NaHCO₃, 5 mM Hepes, pH 7.8). Stage 4-6 oocytes were selected on size, washed in extraction buffer and prepared as for eggs extracts.

For axolotl and *Xenopus* ovary extracts, the ovaries were washed in ice cold extraction buffer and were lysed by using a Dounce homogeneizer, applying 5-10 strokes on ice. The lysate was centrifuged and cryopreserved as described for *Xenopus* egg extracts.

Sturgeon (Sterlet sturgeon; Scientific name: Acipenser ruthenus) extract was prepared as follows: for ovary extracts; the ovaries were washed in ice cold extraction buffer (as for frog egg extracts in example 1) and were lysed by using a Dounce homogeneizer, applying 20 strokes on ice. The lysate was centrifuged and cryopreserved as described for Xenopus egg extracts.

### Cell culture, cell permeabilisation and incubation in extracts

Fibroblasts were isolated from a 35-45 day old bovine foetus or from 12.5-13.5 day old mice and cultured for a maximum of five passages in culture medium (CM: DMEM containing 2 mM glutamine, 0.1 mM β-mercaptoethanol, 2 mM non-essential amino acids, 100 IU ml-1 penicillin and 100 µg ml⁻¹ streptomycin supplemented with 10% FBS) at 39°C for bovine, 37°C for mouse and human, in 5% CO₂. Fibroblasts were isolated from day 13.5 mice (mouse embryonic fibroblasts, MEFs) excluding genital ridges. MEFs were cultured at 37°C in 5% O₂ and CO₂ and used at passage 3 or 4.

Cells were grown to 80% confluence and used for the experiments. After trypsinisation, cells were washed in permeabilisation buffer (PB: 20 mM Hepes, pH 7.3, 110 mM potassium acetate, 5 mM sodium acetate, 2 mM magnesium acetate, 1 mM EGTA, 2 mM DTT, and protease inhibitors), and subsequently incubated in 1 ml of 20-30 µg ml⁻¹ digitonin for 1 minute and 15 seconds on ice (Adam et al (1992) Methods Enzymol. 219, 97-110).

The permeabilisation reaction was stopped by adding 10 ml of PB followed by centrifugation at 700 g for 10 minutes at 4°C. Under these conditions, the plasma membrane permeabilisation rate was 95-100%.

Permeabilised cells were added to oocyte/egg/ovary extracts (1,000 cells µl⁻¹ extract) supplemented with an energy regenerating system (ERS: 150 µg ml-1 creatine phosphokinase, 60 mM phosphocreatine Permeabilised MEFs were added to 20 µl of oocyte extract (either Axolotl, Sturgeon or Xenopus) at 5,000 cells/µl and were incubated for 3 hours, 6 hours or overnight at 15°C. Control cells were incubated with 20 µl of PB.

Following incubation, 0.5 mL of PB was added to rinse the cells which were then pelleted at 3,500 G for 5 minutes. The supernatant was removed, and the pellet was frozen at -80°C until required.

### For the Reverse-Transcription (RT) PCR Reaction

MEF cell pellets were harvested and stored at -80'C. Total RNA was extracted (from ∼100,000 cells) using the RNAeasy system (Qiagen) and DNAse treated (Ambion) before being reverse transcribed using the Superscript III system (Invitrogen). 2ng of RT reaction was used for RT-PCR.

The primers and conditions used are as follows: B-actin: ttctttgcagctccttcgtt, cttttcacggttggccttag (402bp; 56'C annealing, 1min 10secs elongation, 32 cycles). Nanog: atgaagtgcaagcggcagaaa, cctggtggagtcacagagtagttc (464bp; 56'C annealing, 1min 10secs elongation, 35 cycles). Oct-3/4: gtttgccaagctgctgaagc, caccagggtctccgatttgc (238bp; 56'C annealing, 1min 10secs elongation, 38 cycles). The RediTaq system was used for PCRs (Sigma). Mouse ES cell cDNA was used as a positive control, and no RT as negative controls.

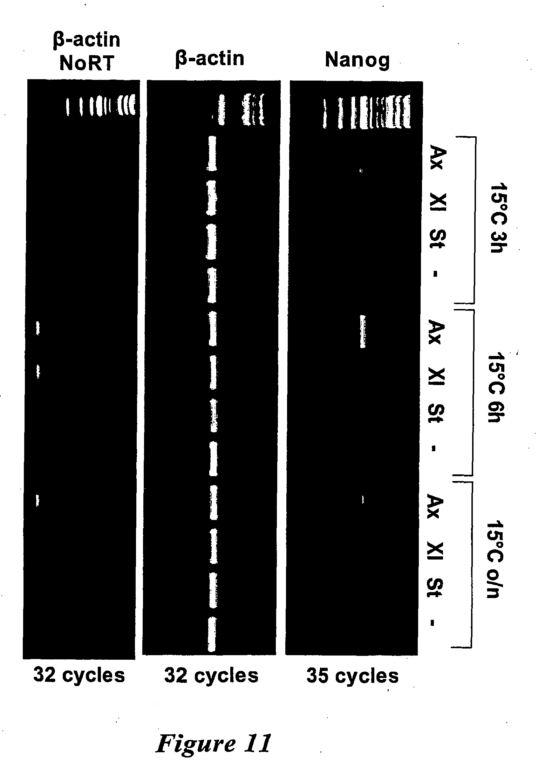

The reaction products were run on a standard 1.2% agarose gel stained with ethidium bromide, and visualized and photographed under UV light (Figure 11). The lanes in the gel correspond to RNA extracted from cells in Axolotl extract (AX); Xenopus Laevis extract (XL); or Sturgeon extract (ST), or cells that were not treated with extract (-); for either 3 hours, 6 hours, or overnight (18 hours).

The left lane shows a 100 bp DNA ladder from Invitrogen, acting as a marker for molecular weight. The top Gel shows the predicted 464 bp DNA band from PCR amplification with nanog primers present in lanes corresponding to cells treated with axolotl or sturgeon extract, but absent in lanes corresponding to cells treated in Xenopus extract, or that were not treated with extract.

The bottom lane shows amplification of mouse β-actin cDNA as a positive control. Note that the predicted 402 bp band is in all lanes as expected, since this gene is not induced by re-programming, it is present in all mouse cells all the time. More cycles of PCR were needed to detect nanog, as expected since this is a regulatory gene and is normally expressed at low levels in cells. β-actin is abundantly expressed, as expected for a structural gene.

In these experiments Oct-4 expression is not activated. This is expected since the Oct-4 promoter is negatively regulated by methylation. Therefore activation requires demethylation of the promoter as a prelude to transcriptional activation, and it is expected that the brief incubation times used in these experiments is not sufficient to demethylate the promoter to completion.

Consistent with this, examples 1 and 5 (injection experiments) show that nanog is relatively easy to activate after injection into the GV of axolotl oocytes. It requires one day for activation. Oct-4 transcription requires about 5 days, presumably reflecting the time needed to demethylate the Oct-4 promoter. Nanog is never activated in cells injected into Xenopus oocytes, even though it does not require prior demethylation.

### Example 3 - Identification of reprogramming using epigenetic marks

Another way at looking for the reprogramming of a differentiated cell to a more pluripotent state is to look at the epigenetic profile of a cell. Epigenetic marks are readily apparent in the complex of DNA and proteins (chromatin) of differentiated cells. In many cases epigenetic marks permanently inactivate the transcription of some genes in a cell. Epigenetic marks are often clustered in large regions of DNA that are tightly compacted into a configuration known as heterochromatin. To achieve reprogramming of a differentiated cell to a pluripotent state requires the reversal of some or all of these repressive epigenetic marks thus making most of the DNA accessible to activation. In this regard, pluripotent cells such as embryonic stem cells contain much less heterochromatin and far fewer epigenetic marks than typical differentiated cells. Presumably this reflects the apparent accessibility of all or most of the genes in pluripotent cells for activation, which is a property assumed essential for pluripotency.

Two epigenetic marks are typically associated with inactive genes. The DNA of most cells contains methyl groups (CH₃) that are covalently linked to cytosine residues in CpG islands. Methylated DNA (CH₃-DNA) is very prominent in heterochromatin, where it can be detected as brightly staining nuclear spots by CH₃-DNA specific antibodies in immuno-chemistry experiments. In addition to CH₃-DNA, a second epigenetic mark is the addition of CH₃ groups to specific residues of histones, the small highly charge nuclear proteins that are bound to DNA to give nuclear DNA a higher order structure referred to as chromatin. Commonly, inactive genes are marked by the presence of CH₃ groups added to the lysine residue 9 (K9) of histone H3, H3K9. In this case the methylated histone (CH₃-histone) residue is bound to the DNA and inhibits its transcriptional activation. In order to reprogram gene expression of cells to a pluripotent state it will be necessary to diminish the levels of CH₃-DNA and CH₃-histones within the differentiated cell nucleus. In this regard, embryonic stem cells contain low levels of CH₃-DNA and CH₃-histone residues.

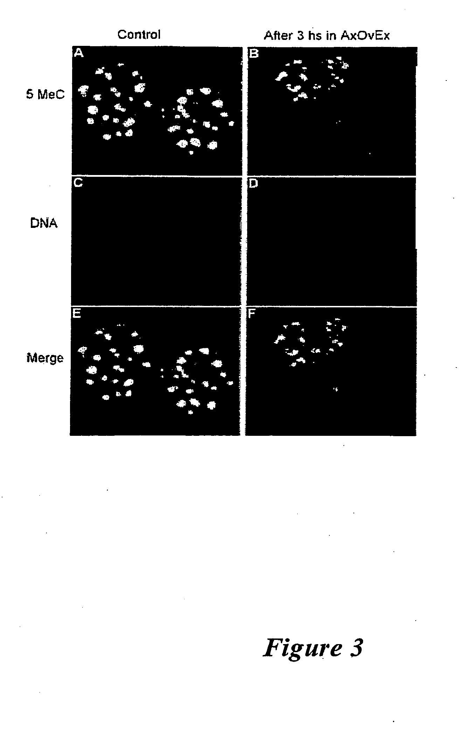

Figure 3 illustrates the ability of extracts prepared from ovaries of axolotls to remove the epigenetic marks present within the chromatin of mouse foetal fibroblasts (PFF). More specifically, Figure 3 illustrates the ability of the extracts to remove DNA methylation. Control untreated cells (A), or cells (B) that were incubated in extract from axolotl ovary (AxOvEx) for 3 hours were analyzed by immunocytochemistry for the presence of CH3-DNA by adding FITC-anti 5-MeC antibody which fluoresces green. However in the black and white figure this can be seen as the bright spots in images A, B, E and F. Control cells (C) and untreated cells (D) were also treated with propidium iodide to assess permeability of the cells in the extracts. Propidium iodide stains red, however in the black and white images it is seen as a dark grey which essentially fills the cells in C and D. Figures E and F show cells stained with both propidium iodide and FITC-anti 5-MeC antibody. PFF incubated in the extract for three hours contain a much reduced level of CH3-DNA staining compared with control cells. The data indicate that extract from axolotl ovary has a powerful ability to demethylate the DNA of PFF, eliminating one of the major epigenetic marks indicative of transcriptionally repressed DNA.

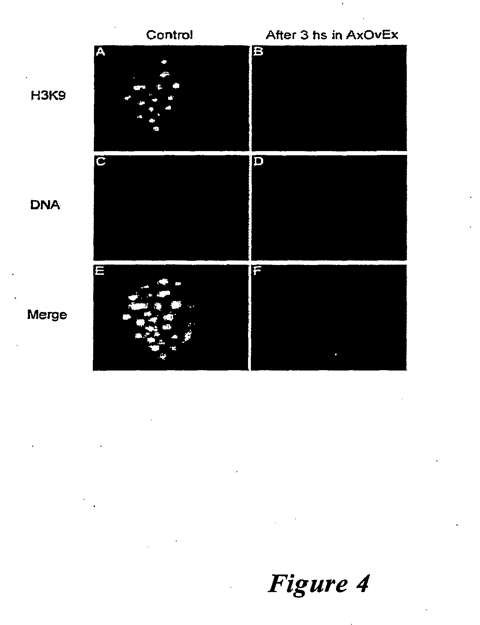

Figure 4 illustrates that the incubation of PFF in axolotl ovary extracts (AxOvEx) eliminates H3K9 staining, thus indicating the removal of epigenetic marks. PFF were incubated in axolotl ovary extract for 3 hours (treated cells). Treated cells and untreated control PFF were analyzed by immunocytochemistry for the presence of H3K9 staining, identifying a major modification of histones that is associated with transcriptional repression. In A (untreated) and B (treated) cells the cells have been incubated with FITC-antiH3K9 antibody which fluoresces green. In the black and white figure this stain can be seen a bright spots. Both groups of cells were also treated with the DNA specific dye DAPI to demonstrate the location of the DNA. This stain emits a blue fluorescence however in the black and white images it can be seen as dark grey, see C and D in Figure 4. Fluorescent images from the H3K9 staining, and DAPI staining were merged for each sample. The data indicate that treatment of PFF in extract from axolotl ovary substantially removes H3K9 staining from the chromatin of PFF, consistent with reprogramming towards a more pluripotent state.

### Example 4 - Cloning of Nanog from primitive animals

Axolotl Nanog full-length cDNA was isolated as follows: Axolotl ovary cDNA was used as a template using degenerate primers F (Forward) (named F1): A (G/C) (C/T) CC (A/G/T) GA (T/C) TCT (G/T) C (C/T) AC (A/T/C) AG (T/C)and R (Reverse) (named R4): C (G/T) (T/C) TGGTT (T/C) TG (A/G) AACCA (C/G) GT (T/C) TT (C/A) (for amino acid sequences N-terminal to the Homeodomain and within the Homeodomain, respectively).

PCR (56'C annealing, 1minute 10seconds elongation, 35 cycles) yielded a ∼260bp fragment.

This fragment was cloned into a plasmid vector and sequenced. Primers were designed for 5' and 3' RACE, using RACE-ready Axolotl ovary cDNA.

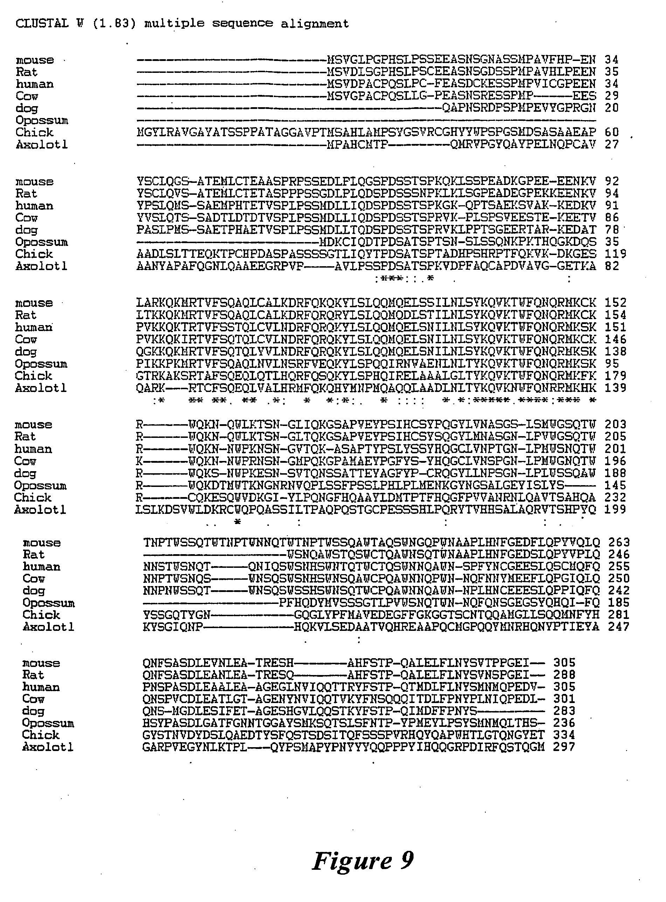

The Smart RACE kit (Clontech) was used and primers and cDNAs were prepared and RACE reactions carried-out according to kit instructions. RACE primers used were: 5' RACE (named RACE-R2): TTCGTCTCACCTCCCACCGCTACAT, 3' RACE (named RACE-F1) CGGCGGATCTCAACCTCACATACAA, along with the corresponding kit primers. 5' RACE yielded a ∼360bp fragment and 3' RACE yielded two fragments of ∼1150bp and ∼750bp, reflecting alternative polyadenylation of Axolotl Nanog cDNA. These products were TA cloned and sequenced. A sequence contig of the 3 overlapping products was constructed giving the full Axolotl Nanog cDNA sequence of ∼1.7kb at its longest. Axolotl nanog amino acid sequence is given in figure 9 compared with nanog sequences from various mammals (nanog has not been isolated from a non-mammalian species before).

### Examples 5 - Reprogramming of human adult cells by axolotl oocytes

Permeabilized BJ human adult primary fibroblasts (from ATCC cell bank collection http://www.1gcpromochem-atcc.com/ **(CR-L-2522))**, were injected as described for mouse experiments in example 1, into the germinal vesicle (nucleus) of axolotl oocytes and the injected oocytes were incubated for 5 days at 18°C.

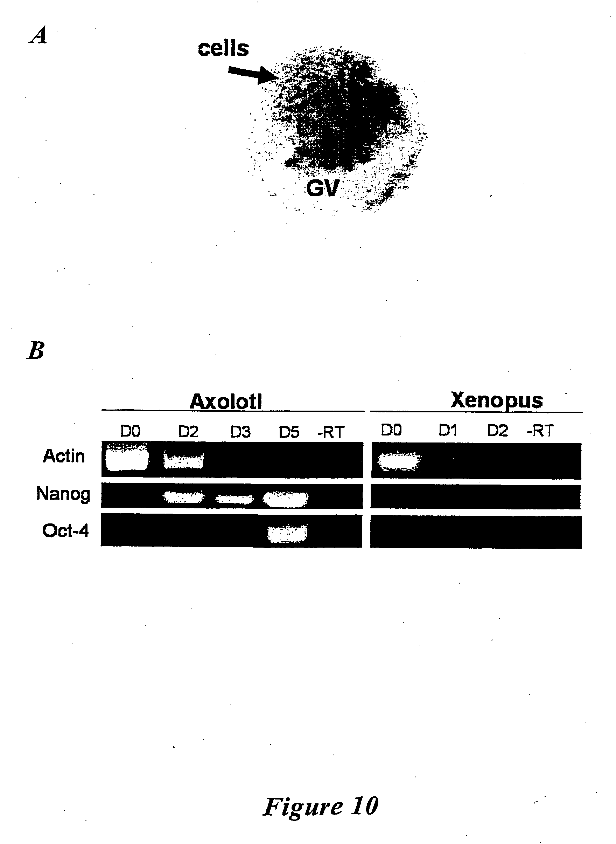

Germinal vesicles were dissected from the oocytes in groups, and those containing human cells were frozen for analysis (Fig 10A).

Total RNA was extracted and DNAse I treated, to avoid genomic DNA contamination, using a commercial extraction kit (RNAeasy kit). RNA extracted from each sample was reverse transcribed to generate complimentary DNA (cDNA).

The cDNA was used for amplification by a standard polymerase chain reaction (PCR) using specific primers designed for the following genes: human Nanog (forward 5' aggcaaacaacccacttctg 3', and reverse 5' tcttcggccagttgtttttc 3'), human POU-5F1 (Oct-4 gene; forward 5' tcccttcgcaagccctcat 3', reverse 5' tgacggtgcagggctccggggaggccccat 3'), and human β-Actin (forward 5' ggacttcgagcaagagatgg 3', and reverse 5' agcactgtgttggcgtacag 3').

As shown in figure 10B, Nanog expression is detected in cells incubated in axolotl oocytes after 48 hrs. In contrast, no Nanog expression is detected in Xenopus injected oocytes. Oct-4 expression is detected in axolotl injected oocytes after 5 days.

Hence, adult human cells (differentiated cells) have been reprogrammed to express the Oct-4 and nanog genes after they were injected into the nucleus of axolotl oocytes. Injection into Xenopus oocytes does not result in activation of nanog.

These results are important because they show that both human and mouse cells can be reprogrammed by the cell /cell extract from a cold blooded vertebrate having the properties of the invention (e.g. primitive vertebrate body plan) and that adult cells can be reprogrammed. Differentiated adult cells are more difficult to reprogram than foetal cells, which are still progressing in their development and may not have had permanent epigenetic marks imposed on the chromatin.

Incubating the injected oocytes at 18 degrees, rather than room temperature presents advantages in the enhanced survivability of the injected oocytes, which can be cultured for at least 5 days which allows sufficient time to trigger activation of Oct-4. Also, as transcription from the mammalian genome is temperature dependent (Alberio et al, 2005) the repression of transcription at this temperature might further aid reprogramming.

### Example 6 -Activation of reporter genes containing nanog binding site

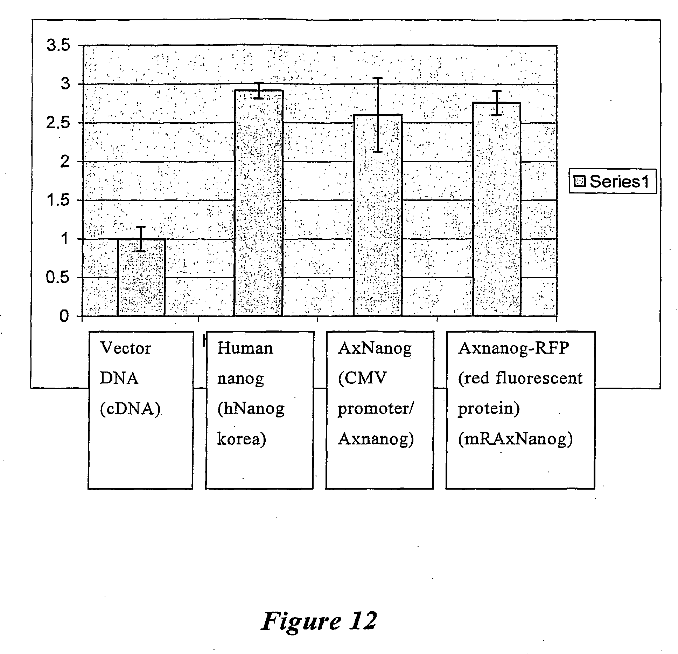

To show that nanog is present it is possible to show that the nanog can activate reporter genes that contain a nanog binding site, such as the promoter of GATA-4. In figure 12 the results of experimental testing of the ability of axolotl nanog (Axnanog) to activate a reporter gene is shown. The GATA-4 promoter is fused to a reporter encoding fire fly luciferase (the protein that makes fire flies glow in the dark. This protein gives off light that can be measured in a machine called a luminometer. The out put can be quantified.

In this experiment empty DNA was compared with human nanog and two clones driving expression of Axnanog.

### Method for figure 12

NIH3T3 cells were seeded into 24 well plates and transfected with 0.25ug/well GATA-4 reporter, 0.05ug/well pTK-RL (Promega) and either empty vector (cDNA), human Nanog, or Axolotl nanog untagged (CMV) or RFP tagged (mR) using Lipofectamine 2000 reagent (Invitrogen). 24hours Post-transfection cells were harvested and processed for Dual luciferase assay (Promega). RLU (relative luciferase units) were ratioed to the empty vector control. Bars show standard deviation; n=3

### Example 7 - Confirmation of isolation of nanog in axolotl

The expression profile of Axnanog was examined and compared to compare the profile of the mouse nanog gene. It was found that Axnanog is expressed exactly when it would be expected to be expressed if it were to play a role in pluripotency during axolotl development.

In mouse embryos the nanog gene is expressed very briefly during early development tin the cells of the inner cell mass and early blastocyst. It is known to play an essential role in the maintenance of pluripotency during this period. To test if Axnanog is expressed during an equivalent period, we analysed its expression in early embryos. A low level of maternal Axnanog RNA is detectable during early and late cleavage stages (EC; LC) then robust expression commences at stage 9, marking the mid-blastula transition, when transcription from the zygotic genome of axolotl embryos commences. Expression peaks in early gastrula, (stage 10), and declines in late gastrula (stage 12). Expression is undetectable in later stages. Thus, Axnanog is expressed during a window of development that coincides with the presence of pluripotent cells, and expression is extinguished after gastrula stages, when all cells are thought to have undergone commitment to a somatic cell lineage, and would not be expected to be pluripotent.

### AxNanog Expression in Early Embryos.

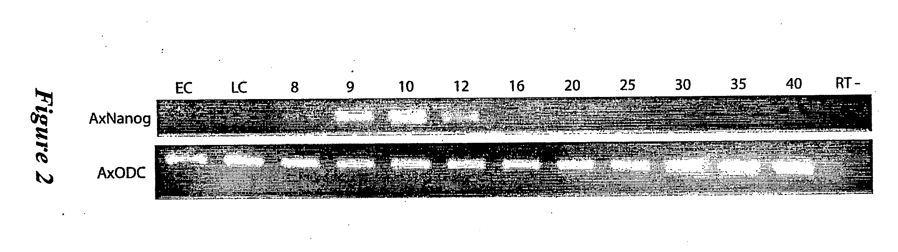

RNA was extracted from 5 axolotl embryos at each of the indicated stages in figure 2 (8, 9, 10, 12, 16, 20, 25, 30, 35 and 40 development stages) (Bordzilovskaya et al; 1989. Developmental stage series of axolotl embryos. In Developmental Biology of the Axolotl. (ed. J. B. Armstrong and G. M. Malacinski), pp. 210-219. New York: Oxford University Press.) using a standard method using Trizol, followed by treatment with DNAse I.

The RNA was reverse transcribed using Superscript (Invitrogen). 0.5 embryo equivalents of cDNA from each stage was used in a PCR with primers for Axnanog (F1: GTTCCAGAACCGAAGGATGA; R1: CGAAGGGTACTGCAGAGGAG 58C, 45 sec, 72 deg. 1 min) or Axolotl ornithine decarboxylase (ODC; Fl: TGCGTTGGTTTAAAGCTCTC; R1: ACATGGAAGCTCACACCAAT 56 C 45 sec; 72 C 1 min), a constitutively expressed gene used a positive control for cDNA integrity. PCR was for 35 cycles (Axnanog) or 30 cycles (ODC).

The reaction products were separated on a 1.2% agarose gel containing ethidium bromide, then photographed under UV light (figure 2).

### Example 8 - DNA demethylation confirmation

The global DNA demethylation was confirmed by flow cytometry using the protocol described previously (Habib et al., (1999). Exp.Cell Res. 249, 46-53.) with slightly modifications.

Cells in suspension were submitted to successive low-speed (300g) pelleting and gentle resuspension steps in washed twice with phosphate buffered saline (PBS) supplemented with 0.1% tween 20 and 1% bovine serum albumin (BSA) and then were fixed with 9 vol of methanol/ PBS (88% methanol/12% PBS vol/vol) refrigerated at -20°C for 20 minutes. After two washes with PBST-BSA at room temperature cells were treated successively with 2 N HCl at 37°C for 30 minutes, then with Tris HCL buffer (pH 8.8) for 5 min. Then cells were treated for 1 hour at 37°C with a blocking solution made of PBS-T supplemented with 5% BSA, following the incubation with anti-5-MeC antibody (where) at 4°C over night.

Cells were then successively rinsed three times with PBS-T and incubated for 1 hour at room temperature with rabbit anti-mouse immunoglobulin conjugated to fluorescein isothiocyanate (Dako, Trappes, France) diluted 1 in 200 in PBST-BSA. Finally samples were washed three times with PBS and were stained with propidium iodide (PI) (50 mg/ml in PBS) for 30 min before flow cytometry. Analyses were performed by Epics XL flow cytometer (Beckman Coulter).

Similar protocols were used for detecting the changes of H3K9 and HP1 alpha by flow cytometry. Briefly, cells were washed twice with pH 7.4 PBS supplemented with 1% BSA and 0.1% Tween 20 (PBST-BSA) and then were fixed with 9 vol of methanol/ PBS (88% methanol/12% PBS vol/vol) refrigerated at -20°C for 20 minutes.

Then cells were treated for 1 hour at 37°C with a blocking solution made of PBS-T supplemented with 5% BSA, following the incubation with anti-Tri methylated H3K9 or anti-HP1 alpha antibody (where) at 4°C over night. Cells were then successively rinsed three times with PBS-T and incubated for 1 hour at room temperature with Dunkey anti rabbit (1:100, Juckson USA) or goat anti-mouse immunoglobulins conjugated to fluorescein isothiocyanate (Dako, Trappes, France) diluted 1 in 200 in PBST-BSA respectively.

Finally samples were washed three times with PBS and were stained with propidium iodide (PI) (50 mg/ml in PBS) for 30 min before flow cytometry.

Analyses were performed by Epics XL flow cytometer (Beckman Coulter).

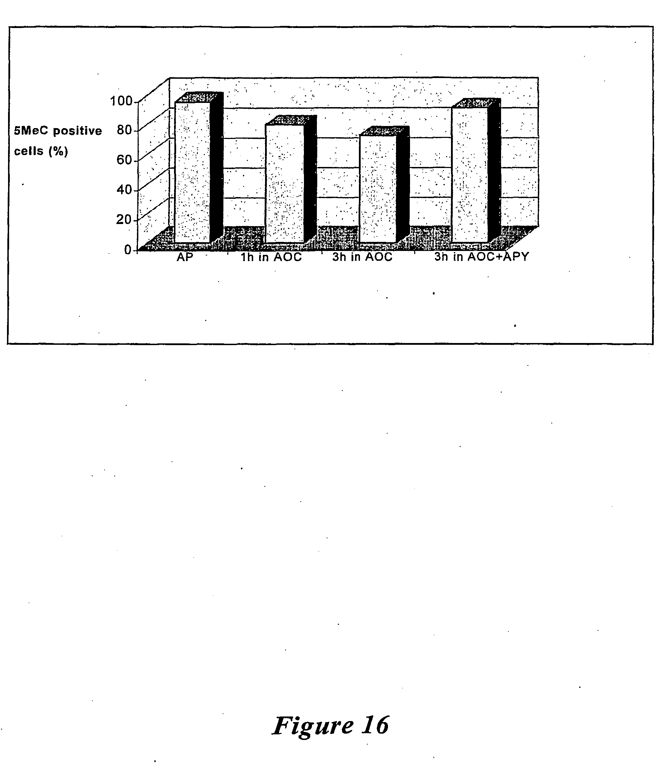

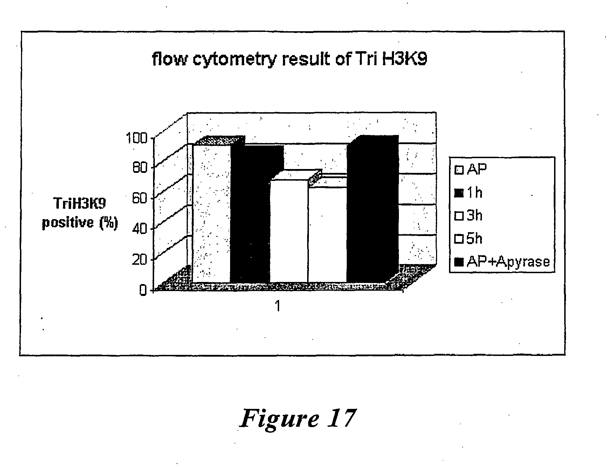

The results of the flow cytometry are given in figures 16 and 17.

### Discussion

The cells were sorted based on the flourescence staining with the CH3-DNa antibody, and the histone H3K9 antibody, and then examined for what percentage of the cells have the full control level of staining, and what percentage show diminished fluorescence over time.

As the figures show, the overall level of fluorescence in the cell, in both experiments is reduced. This confirms the earlier results and demonstrates that loss of methylated DNA and Histone H3K9 is being achieved.

### Example 9 - Use of axolotl oocyte extract in enhancing sheep cloning

The cells and cell extracts of the invention may be used in enhancing cloning procedures such as sheep cloning by incubating the cells with Axolotl oocyte extracts prior to nuclear transfer (NT).

Sheep fibroblasts were permeabilized as described for bovine cells by Alberio et al., (2005). The cells were incubated in axolotl oocyte extracts at 18°C for 3 hrs and then used as nuclear donors for NT. Permeabilized cells are normally easily recognised under a microscope with a swollen and round shape and were therefore chosen for NT. The nuclear transfer procedure was carried out in the same manner as described by Lee et al., (2006) Biol Reprod. 74:691-8.

After 7 days in culture development to blastocyst was assessed under a microscope. Five cloned embryos developed to blastocyst after 7 days, indicating that cells incubated in axolotl extracts are viable donors for NT.

This experiment has shown that exposure of mammalian somatic cells to axolotl oocyte extracts is not detrimental for preimplantation development of mammalian cloned embryos.

It is expected that these embryos will show an improvement in the development to term of cloned embryos made with cells exposed to extracts of the invention. Gestation and development of the embryos is ongoing.

### Example 10 - Kit for reprogramming

The cells and cell extracts of the inventions can be provided in the form of a kit for use in the method of the invention. These kits preferably contain the cell/cell extract reagent, and at least one of the following:

Instructions for use; progenitor medium for re-differentiation of the reprogrammed cells; disposable equipment for conducting the re-Programming e.g. multi-well plates, dispensers such as pre-loaded pipettes; differentiated cells to be reprogrammed and permeabilization Buffer.

The kits may be customised according to the re-differentiated cell type is required in that the progenitor medium and the instructions may vary according to the cell type and end use.

## Claims

1. A method of producing a reprogrammed cell or reprogrammed cell nucleus, comprising exposing a permeabilised differentiated cell, or the nucleus of a permeabilised differentiated cell, at a temperature of between 5°C and 30°C, to a cell or cell extract thereof derived from an oocyte, egg, ovary or early embryo of a cold blooded vertebrate, wherein the cold blooded vertebrate is an axolotl or a sturgeon.

2. A method according to claim 1 where the reprogrammed cell is an embryonic stem cell-like cell.

3. A method according to any previous claim where the reprogrammed cell, and/or the reprogrammed cell nucleus, expresses Oct-4.

4. A method according to any previous claim where the reprogrammed cell, or the reprogrammed cell nucleus, expresses nanog.

5. A method according to any preceding claim wherein the reprogrammed cell, or the reprogrammed cell nucleus is pluripotent.

6. A method according to any preceding claim wherein the Oct-4 and/or the nanog in the cold-blooded vertebrate oocyte, egg, ovary or early embryo cell or cell extract has at least 69% amino acid identity with the human transcription factor Oct-4 and the human nanog protein, respectively.

7. A method according to any previous claim wherein the Oct-4 and/or the nanog in the cold blooded vertebrate oocyte, egg, ovary or early embryo cells has at least 69% amino acid identity with the DNA binding domains (DBD) of the human transcription factor Oct-4 or the human nanog protein, respectively.

8. A method according to any preceding claim wherein the oocyte, egg, or early embryo cell extract comprises material from the nucleus or germinal vesicle (GV) of the oocyte, egg, or early embryo cell.

9. A method according to any preceding claim wherein the differentiated cell is a eukaryotic cell.

10. A method according to claim 9 wherein the differentiated cell is mammalian.

11. A method according to claim 10 wherein the differentiated cell is human.

12. A method of producing a re-differentiated cell comprising:
(a) producing a reprogrammed cell as described in any of claims 1 to 11; and
(b) re-differentiating the reprogrammed cell into a differentiated cell of the same type, or a different type, to the differentiated cell from which it is derived.

13. A method as claimed in claim 12 wherein the re-differentiation is effected using a progenitor medium.

14. A kit for reprogramming a differentiated cell, or for reprogramming the nucleus of a differentiated cell, comprising a cell or cell extract thereof derived from an oocyte, egg, ovary or early embryo of a cold blooded vertebrate, wherein the cold blooded vertebrate is an axolotl or a sturgeon;
instructions to use the oocyte, egg, ovary or early embryo, or extract thereof, and a progenitor medium to effect the further steps of re-differentiation of the reprogrammed cell.

15. A kit as claimed in claim 14 further comprising one or more differentiated cells to be reprogrammed.

## Patentansprüche

1. Verfahren zur Herstellung einer reprogrammierten Zelle oder eines reprogrammierten Zellkerns, umfassend das Einwirken einer Zelle oder eines Zellextrakts derselben, die von einem Oozyt, Ei, Ovar oder frühen Embryo eines Kaltblüter-Wirbeltiers stammen, auf eine permeabilisierte differenzierte Zelle oder den Kern einer permeabilisierten differenzierten Zelle bei einer Temperatur zwischen 5°C und 30°C, wobei das Kaltblüter-Wirbeltier ein Axolotl oder ein Stör ist.

2. Verfahren nach Anspruch 1, wobei die reprogrammierte Zelle eine einer embryonalen Stammzelle ähnliche Zelle ist.

3. Verfahren nach einem der vorhergehenden Ansprüche, wobei die reprogrammierte Zelle und/oder der reprogrammierte Zellkern Oct-4 exprimiert.

4. Verfahren nach einem der vorhergehenden Ansprüche, wobei die reprogrammierte Zelle oder der reprogrammierte Zellkern NANOG exprimiert.

5. Verfahren nach einem der vorhergehenden Ansprüche, wobei die reprogrammierte Zelle oder der reprogrammierte Zellkern pluripotent ist.

6. Verfahren nach einem der vorhergehenden Ansprüche, wobei das Oct-4 und/oder das NANOG in der Zelle oder dem Zellextrakt von einem Oozyt, Ei, Ovar oder frühen Embryo eines Kaltblüter-Wirbeltiers mindestens 69% Aminosäure-Identität mit dem humanen Transkriptionsfaktor Oct-4 bzw. dem humanen NANOG-Protein aufweisen.

7. Verfahren nach einem der vorhergehenden Ansprüche, wobei das Oct-4 und/oder das NANOG in den Zellen von einem Oozyt, Ei, Ovar oder frühen Embryo eines Kaltblüter-Wirbeltiers mindestens 69% Aminosäure-Identität mit den DNA-bindenden Domänen (DBD) des humanen Transkriptionsfaktors Oct-4 bzw. des humanen NANOG-Proteins aufweisen.

8. Verfahren nach einem der vorhergehenden Ansprüche, wobei der Zellextrakt von einem Oozyt, Ei oder frühen Embryo Material von dem Kern oder Germinalvesikel (GV) der Zelle von einem Oozyt, Ei oder frühen Embryo umfasst.

9. Verfahren nach einem der vorhergehenden Ansprüche, wobei die differenzierte Zelle eine eukaryotische Zelle ist.

10. Verfahren nach Anspruch 9, wobei die differenzierte Zelle eine Säugerzelle ist.

11. Verfahren nach Anspruch 10, wobei die differenzierte Zelle eine Humanzelle ist.

12. Verfahren zur Herstellung einer redifferenzierten Zelle, umfassend:
(a) Herstellen einer reprogrammierten Zelle gemäß der Beschreibung in einem der Ansprüche 1 bis 11 und
(b) Redifferenzieren der reprogrammierten Zelle in eine differenzierte Zelle der gleichen Art oder einer unterschiedlichen Art gegenüber der differenzierten Zelle, von der sie stammt.

13. Verfahren nach Anspruch 12, wobei die Redifferenzierung unter Verwendung eines Vorläufermediums bewirkt wird.

14. Kit zur Reprogrammierung einer differenzierten Zelle oder zur Reprogrammierung des Kerns einer differenzierten Zelle, umfassend eine Zelle oder einen Zellextrakt hiervon, die von einem Oozyt, Ei, Ovar oder frühen Embryo eines Kaltblüter-Wirbeltiers stammen, wobei das Kaltblüter-Wirbeltier ein Axolotl oder ein Stör ist;
Anweisungen zur Verwendung von dem Oozyt, Ei, Ovar oder frühen Embryo oder einem Extrakt hiervon, und ein Vorläufermedium zum Durchführen der weiteren Stufen einer Redifferenzierung der reprogrammierten Zelle.

15. Kit nach Anspruch 14, das ferner eine oder mehrere differenzierte Zellen zur Reprogrammierung umfasst.

## Revendications

1. Procédé de production d'une cellule reprogrammée ou d'un noyau de cellule reprogrammée, comprenant l'exposition d'une cellule différentiée perméabilisée, ou du noyau d'une cellule différentiée perméabilisée, à une température comprise entre 5 °C et 30 °C, à une cellule ou un extrait cellulaire de celle-ci dérivé(e) d'un oocyte, d'un oeuf, d'un ovaire ou d'un jeune embryon d'un vertébré à sang froid, dans lequel le vertébré à sang froid est un axolotl ou un esturgeon.

2. Procédé selon la revendication 1, dans lequel la cellule reprogrammée est une cellule de type cellule souche embryonnaire.

3. Procédé selon l'une quelconque des revendications précédentes, dans lequel la cellule reprogrammée et/ou le noyau de cellule reprogrammée exprime Oct-4.

4. Procédé selon l'une quelconque des revendications précédentes, dans lequel la cellule reprogrammée ou le noyau de cellule reprogrammée exprime nanog.

5. Procédé selon l'une quelconque des revendications précédentes, dans lequel la cellule reprogrammée ou le noyau de cellule reprogrammée est pluripotent(e).

6. Procédé selon l'une quelconque des revendications précédentes, dans lequel le Oct-4 et/ou le nanog dans la cellule ou l'extrait cellulaire d'oocyte, d'oeuf, d'ovaire ou de jeune embryon de vertébré à sang froid a au moins 69 % d'identité des acides aminés avec le facteur de transcription humain Oct-4 et la protéine nanog humaine, respectivement.

7. Procédé selon l'une quelconque des revendications précédentes, dans lequel le Oct-4 et/ou le nanog dans les cellules d'oocyte, d'oeuf, d'ovaire ou jeune embryon de vertébré à sang froid a au moins 69 % d'identité des acides aminés avec les domaines de liaison à l'ADN (DBD) du facteur de transcription humain Oct-4 ou de la protéine nanog humaine, respectivement.

8. Procédé selon l'une quelconque des revendications précédentes, dans lequel l'extrait cellulaire d'oocyte, d'oeuf ou de jeune embryon comprend un matériau du noyau ou de la vésicule germinale (GV) de la cellule d'oocyte, d'oeuf ou de jeune embryon.

9. Procédé selon l'une quelconque des revendications précédentes, dans lequel la cellule différentiée est une cellule eucaryote.

10. Procédé selon la revendication 9, dans lequel la cellule différentiée est de mammifère.

11. Procédé selon la revendication 10, dans lequel la cellule différentiée est humaine.

12. Procédé de production d'une cellule re-différentiée, comprenant :
(a) la production d'une cellule reprogrammée selon l'une quelconque des revendications 1 à 11 ; et
(b) la re-différentiation de la cellule reprogrammée en une cellule différentiée du même type, ou d'un type différent, par rapport à la cellule différentiée dont elle est dérivée.

13. Procédé selon la revendication 12, dans lequel la re-différentiation est effectuée en utilisant un milieu progéniteur.

14. Kit pour reprogrammer une cellule différentiée, ou pour reprogrammer le noyau d'une cellule différentiée, comprenant une cellule ou un extrait cellulaire de celle-ci dérivé(e) d'un oocyte, d'un oeuf, d'un ovaire ou d'un jeune embryon d'un vertébré à sang froid, dans laquelle le vertébré à sang froid est un axolotl ou un esturgeon ; des instructions pour utiliser l'oocyte, l'oeuf, l'ovaire ou le jeune embryon, ou un extrait de ceux-ci, et un milieu progéniteur pour mettre en oeuvre les étapes supplémentaires consistant à re-différentier la cellule reprogrammée.

15. Kit selon la revendication 14, comprenant en outre une ou plusieurs cellules différentiées devant être reprogrammées.
